# EUROPEAN PATENT SPECIFICATION

(11) **EP 4 212 219 B1**
(45) Date of publication and mention of the grant of the patent: **02.09.2026**
(21) Application number: 20958622.1
(22) Date of filing: 20.10.2020
(51) Int. Cl.: A63B 69/00, A63B 71/06, G06F 3/0482, G16H 20/30, A61B 5/11, A61B 5/00, A61B 5/103

(54) **EXERCISE IMPROVEMENT INSTRUCTION DEVICE, EXERCISE IMPROVEMENT INSTRUCTION METHOD, AND EXERCISE IMPROVEMENT INSTRUCTION PROGRAM**
ÜBUNGSVERBESSERUNGSANWEISUNGSVORRICHTUNG, ÜBUNGSVERBESSERUNGSANWEISUNGSVERFAHREN UND ÜBUNGSVERBESSERUNGSANWEISUNGSPROGRAMM
DISPOSITIF D'INSTRUCTION POUR L'OPTIMISATION D'EXERCICE, PROCÉDÉ D'INSTRUCTION POUR L'OPTIMISATION D'EXERCICE ET PROGRAMME D'INSTRUCTION POUR L'OPTIMISATION D'EXERCICE

(43) Date of publication of application: 19.07.2023
(73) Proprietor: ASICS Corporation, Kobe-shi Hyogo 650-8555 (JP)
(72) Inventor: HIRAKAWA Nao, Kobe-shi Hyogo 650-8555 (JP); INOMATA Takashi, Kobe-shi Hyogo 650-8555 (JP); MORI Hiroto, Kobe-shi Hyogo 650-8555 (JP); ICHIKAWA Masaru, Kobe-shi Hyogo 650-8555 (JP); NISHIMURA Noriko, Kobe-shi Hyogo 650-8555 (JP)
(74) Representative: Marks & Clerk LLP
(86) International application number: PCT/JP2020/039390
(87) International publication number: WO 2022/085069

(56) References cited:
- WO-A1-2019/008771
- WO-A1-2020/100671
- JP-A- 2017 000 167
- JP-A- 2018 143 537
- JP-A- 2018 143 537
- JP-B1- 6 644 290
- US-A1- 2018 104 541
- US-A1- 2018 220 937
- US-A1- 2019 231 223
- US-B2- 9 656 119

## Description

### TECHNICAL FIELD

The present invention relates to a technology for identifying problems for improvement of exercise motions.

### BACKGROUND ART

In recent years, mobile communication terminals such as smartphones have been becoming more lightweight, and so-called wearable devices have been actively developed, as typified by smartwatches. These devices can be worn also during exercise, such as running, and can measure exercise motions with built-in acceleration sensors and the like. There are also services and applications that provide guidance for improving exercise motions based on the measurement results. US 2019/231223 describes a compensatory movement source body region identification method including: a step of first determination that determines existence of the compensatory movement in a first motion based on whether or not first measurement data satisfies a first criterion; and/or a step of second determination that determines existence of the compensatory movement in a second motion that is of lower complicity than the first motion based on whether or not second measurement data satisfies a second criterion; and a step of identifying at least one or more body region to be the source for causing the compensatory movement based on whether or not third measurement data in a third motion that is of lower complicity than the second motion satisfies a third criterion, when determined in the step of first determination and/or the step of second determination that there is the compensatory movement. JP 2018 143537 A describes a motion analysis device, a motion analysis system, a motion analysis method, and a motion analysis program.

### PRIOR ART REFERENCE

### PATENT LITERATURE

Patent Literature 1: Japanese Unexamined Patent Application Publication No. 2014-124448

### SUMMARY OF INVENTION

### TECHNICAL PROBLEM

As devices have been becoming smarter in recent years, the types of built-in sensors have been increasing. Also, since each sensor has been becoming more sophisticated, a large amount of measurement data can be obtained through a single exercise. By analyzing such measurement data, a number of problems in exercise motions can be extracted from various perspectives. However, among the problems, it is difficult to identify a problem that leads to effective improvement of the exercise motions.

The present invention has been made in view of such a situation, and a purpose thereof is to provide an exercise improvement guidance device that can identify a problem that leads to effective improvement of exercise motions.

### SOLUTION TO PROBLEM

Aspects of the invention are in accordance with the appended independent claims. Various optional features are the subject of the dependent claims. There is described an exercise improvement guidance device that includes: a hierarchical index storage unit that stores hierarchized indexes for improvement of an exercise motion; and a problem index identification unit that evaluates the hierarchized indexes based on measurement data during an exercise and identifies a problem index.

In the device described in the preceding paragraph aspect, the hierarchized indexes are evaluated based on measurement data during an exercise, and a problem index is identified. By referring to the relationships among the indexes shown in the hierarchical structure, a problem index that leads to effective improvement of exercise motions can be identified.

There is also described an exercise improvement guidance method that includes: a hierarchical index storing step of storing hierarchized indexes for improvement of an exercise motion; and a problem index identification step of evaluating the hierarchized indexes based on measurement data during an exercise and identifying a problem index.

There is also described an exercise improvement guidance program that causes a computer to implement: a hierarchical index storing step of storing hierarchized indexes for improvement of an exercise motion; and a problem index identification step of evaluating the hierarchized indexes based on measurement data during an exercise and identifying a problem index.

Optional combinations of the aforementioned constituting elements, and implementation of the present invention in the form of methods, apparatuses, systems, recording media, and computer programs may also be practiced as additional modes of the present invention.

### ADVANTAGEOUS EFFECTS OF INVENTION

The present invention enables identification of a problem that leads to effective improvement of exercise motions.

### BRIEF DESCRIPTION OF DRAWINGS

FIG. 1 is an overall configuration diagram of a system that includes an exercise improvement guidance device according to an embodiment.
FIG. 2 is a diagram that shows an example of a hierarchical structure of indexes stored in a hierarchical index storage unit.
FIG. 3 is a flow diagram of exercise improvement guidance processing performed by the exercise improvement guidance device using the hierarchical structure.
FIG. 4 is a flow diagram of exercise improvement guidance processing performed by the exercise improvement guidance device when a user performs another running.
FIG. 5 is a diagram that shows another example of the hierarchical structure in the form of a directed graph.

### DESCRIPTION OF EMBODIMENTS

In the present embodiment, guidance is provided to improve exercise motions in running as an example, such as the running form and how to use force in running. Indexes for improvement of exercise motions are hierarchized, and indexes in the highest layer include the "landing impact", "vertical motion", "braking force", and the like. Each of these indexes in the highest layer is subdivided stepwise toward the lower layers, and, in the lowest layer, there are indexes that can be improved individually, such as the "ground contact position", "ground contact angle", "trunk angle", and the like. By searching such a hierarchical structure of indexes from an upper layer toward a lower layer with reference to measurement data obtained during running, a problem index that should be preferentially improved can be identified.

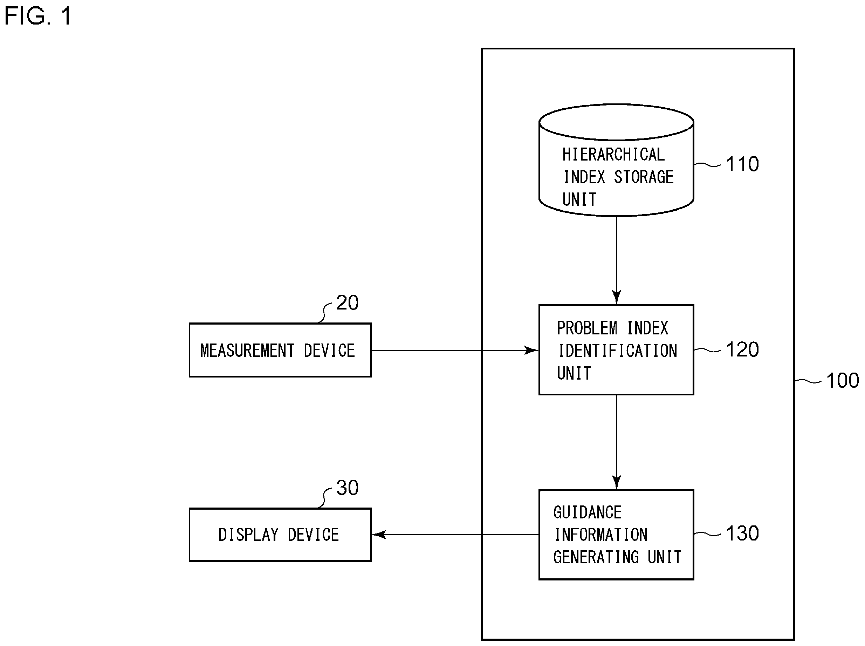

FIG. 1 is an overall configuration diagram of a system that includes an exercise improvement guidance device 100 according to the embodiment. The exercise improvement guidance device 100 generates guidance information for improving exercise motions in running based on measurement data measured by a measurement device 20 while a user is running. The guidance information thus generated is displayed on a display device 30 used by the user.

The measurement device 20 may be a wearable device, such as a smartwatch, or a smartphone that can be worn by the user during running, for example, and measures exercise motions during running with a built-in sensor. However, the measurement device 20 in the present embodiment is not limited thereto and may be any device that has a data measurement function during running and a minimum data transmission function to transmit the measurement data to the exercise improvement guidance device 100. For example, as the measurement device 20, a camera (an image capturing device) for capturing images of the user during running may be used. In this case, the data captured by the camera is provided as the measurement data to the exercise improvement guidance device 100.

In this way, since various devices can be used as the measurement device 20, the exercise improvement guidance device 100 can acquire various measurement data associated with the user's exercise motions during running.

For example, when a wearable device or a smartphone is used as the measurement device 20 as described above, through an acceleration sensor, an angular velocity sensor, a position sensor (such as the GPS), a magnetic sensor, and the like built into the device, basic physical quantities associated with the user's position and the exercise performed by the user can be obtained as the measurement data. By combining such measurement data as appropriate and performing analysis, not only basic information, such as the user's position, speed, and acceleration during running, but also information on the details of the user's exercise motions, such as the user's posture, strength and angle of pushing off, and position and angle of ground contact during running, can be obtained. In addition, information regarding the running course, such as the distance, altitude, and gradient, can also be obtained.

Furthermore, when the measurement device 20 is equipped with a sensor that measures the external environment, such as an ambient light sensor that measures brightness and the like, a temperature sensor, and a humidity sensor, the exercise improvement guidance device 100 can provide appropriate exercise improvement guidance also in consideration of the external environment during running. Also, when a wearable device capable of measuring biological signals, such as heartbeat, is used as the measurement device 20, which has been actively developed in recent years, the exercise improvement guidance device 100 can provide appropriate exercise improvement guidance also in consideration of the user's physical condition.

The measurement device 20 need not necessarily be worn by the user during exercise and may measure the user during exercise from the surroundings. The image capturing by a camera as described previously is considered to be a typical example, but the device is not limited thereto. For example, when the user is running in a restricted indoor area using a treadmill or the like, the measurement device 20 that is difficult to carry can also be used, so that the variety of available measurement data is remarkably increased.

The various measurement devices 20 described above with examples may be used individually or may be used in combination. For example, a wearable device as a first measurement device 20 may be worn by the user to perform measurement, and a camera as a second measurement device 20 may be used to capture images of the user. Based on the measurement data from such multiple measurement devices 20, the exercise improvement guidance device 100 can analyze the user's exercise motions from multiple aspects and generate guidance information for effective improvement of the exercise motions.

The display device 30 is a device that displays the guidance information for improving exercise motions, which has been generated by the exercise improvement guidance device 100 based on the measurement data from the measurement device 20. For example, when a smartphone is used as the measurement device 20, it also functions as the display device 30 that displays the guidance information. Also, when a camera or the like that does not have a function to display the guidance information is used as the measurement device 20, another device owned by the user, such as a smartphone, tablet, or personal computer, is used as the display device 30. The means for presenting the guidance information for improving exercise motions, which has been generated by the exercise improvement guidance device 100 based on the measurement data from the measurement device 20, is not limited to the display by the display device 30. For example, the guidance information described above may be presented to the user by outputting voice from a voice device.

The exercise improvement guidance device 100 is configured on a server capable of communicating with the measurement device 20 and the display device 30 via a wide area communication network such as the Internet. The exercise improvement guidance device 100 includes a hierarchical index storage unit 110, a problem index identification unit 120, and a guidance information generating unit 130. Meanwhile, the present embodiment is not limited to the above, and the exercise improvement guidance device 100 may be configured to be capable of communicating with the measurement device 20 and the display device 30 via a local communication network such as a LAN. Also, when the measurement device 20 or the display device 30 is an information processing device, such as a smartphone, tablet, or personal computer, the functions of the exercise improvement guidance device 100 may be implemented as application software running on the information processing device. Also, the data measured by the measurement device 20 may be stored in a storage medium, such as a USB flash drive, and may be read by a personal computer or the like equipped with the functions of the exercise improvement guidance device 100 so as to provide the exercise improvement guidance in the present embodiment.

The hierarchical index storage unit 110 stores hierarchized indexes for improvement of exercise motions, such as the running form and how to use force in running. The hierarchical structure of indexes stored in the hierarchical index storage unit 110 is configured during the initial setup of the system, for example. The hierarchical structure thus configured can be used for multiple exercise sessions without being updated for a certain period of time, in order to provide consistent exercise improvement guidance. Also, the hierarchical structure may be updated by adding a new index or by changing or deleting an existing index. Further, teacher data showing a correlation between the performance of an exercise motion and each index may be obtained, and, based on the teacher data, a learning model into which the performance of an exercise motion is input and from which a corresponding index is output may be generated by machine learning. With such a learning model, an index that leads to effective improvement of exercise motions can be preferentially set, based on the actual performance of the exercise motions. Furthermore, by autonomously analyzing a term used in each index and its biomechanical meaning, the hierarchical structure of indexes can be automatically generated.

Based on the hierarchical structure of indexes stored in the hierarchical index storage unit 110, the problem index identification unit 120 identifies a problem index with reference to measurement data during running measured by the measurement device 20. In specific, the problem index identification unit 120 evaluates the hierarchized indexes based on the measurement data during running to identify the problem index.

The search for the problem index by the problem index identification unit 120 is performed from an upper layer toward a lower layer of the hierarchical structure. At the time, among multiple indexes in the same layer of the hierarchical structure, an index of which the divergence between predetermined reference data and the measurement data is largest is selected, and the search for the problem index is performed therefrom toward a further lower layer. The problem index identification unit 120 then identifies the problem index in the lowest layer of the hierarchical structure. In the embodiment, the implementation is not limited to starting the search from the highest layer of the hierarchical structure. For example, the user may specify in advance an arbitrary index to be improved, and the search for the problem index may be performed from the layer of the arbitrary index toward the lower layer.

The guidance information generating unit 130 generates guidance information for improving the problem index identified by the problem index identification unit 120. The guidance information thus generated is transmitted to the display device 30 and displayed on the display screen thereof.

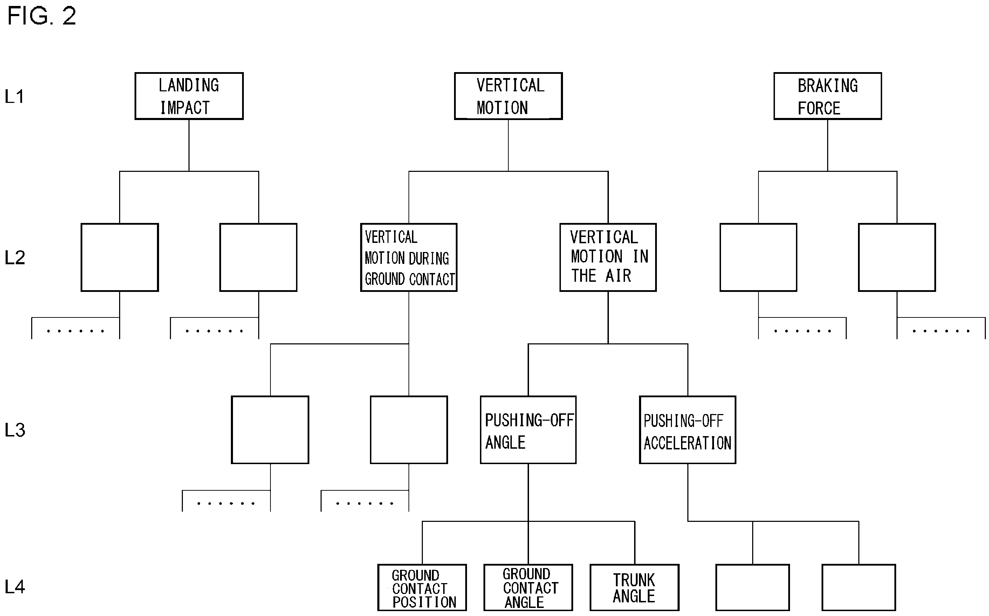

FIG. 2 shows an example of the hierarchical structure of indexes stored in the hierarchical index storage unit 110. The hierarchical structure is constituted by four layers, from a first layer L1 as the highest layer to a fourth layer L4 as the lowest layer. In the first layer L1, the "landing impact", "vertical motion", and "braking force", which are major indexes used to evaluate running exercise motions, are illustratively arranged.

In a second layer L2 immediately below the first layer L1, indexes resulting from subdivision of each index in the first layer L1 are arranged. For example, the "vertical motion" in the first layer L1 is subdivided into two indexes of "vertical motion during ground contact" and "vertical motion in the air" in the second layer L2. More specifically, problems with the "vertical motion" in the first layer L1 fall roughly into problems with vertical motions during ground contact and problems with vertical motions in the air; accordingly, indexes corresponding to the respective cases are provided in the second layer L2 so that the cause of the problem can be specifically identified. Similarly, each of the other indexes of the "landing impact" and "braking force" in the first layer L1 is subdivided in the second layer L2 and lower, though the illustration and explanation thereof is omitted here. When an index in an upper layer is subdivided into indexes in a lower layer, strict logic is not required, so that multiple lower indexes may overlap each other, or the combination of multiple lower indexes may not completely reproduce the upper index.

In a third layer L3 immediately below the second layer L2, indexes resulting from subdivision of each index in the second layer L2 are arranged. For example, the "vertical motion in the air" in the second layer L2 is subdivided into two indexes of the "pushing-off angle" and "pushing-off acceleration" in the third layer L3. More specifically, problems with the "vertical motion in the air" in the second layer L2 fall roughly into problems with the pushing-off angle at takeoff and problems with the pushing-off acceleration at takeoff; accordingly, indexes corresponding to the respective cases are provided in the third layer L3 so that the cause of the problem can be specifically identified. Similarly, the other index of the "vertical motion during ground contact" in the second layer L2 is subdivided in the third layer L3 and lower, though the illustration and explanation thereof is omitted here.

In the fourth layer L4 immediately below the third layer L3, indexes resulting from subdivision of each index in the third layer L3 are arranged. For example, the "pushing-off angle" in the third layer L3 is subdivided into three indexes of the "ground contact position", "ground contact angle", and "trunk angle" in the fourth layer L4. More specifically, problems with the "pushing-off angle" in the third layer L3 fall roughly into problems with the ground contact position, problems with the ground contact angle, and problems with the trunk angle; accordingly, indexes corresponding to the respective cases are provided in the fourth layer L4 so that the cause of the problem can be specifically identified. Similarly, the other index of the "pushing-off acceleration" in the third layer L3 is subdivided in the fourth layer L4, though the illustration and explanation thereof is omitted here.

In the hierarchical structure as described above, by sequentially evaluating each index from an upper layer toward a lower layer with reference to the measurement data obtained during running, a problem index to be improved can be specifically identified. For example, when there is a problem with the "vertical motion" in the first layer L1, which is the highest layer, if the specific cause of the problem is not identified, appropriate exercise improvement guidance cannot be provided. With the hierarchical structure of the present embodiment, however, when a problem of "vertical motion" in an upper layer is identified, the search for the specific cause can be conducted therefrom toward the lower layer. Thereafter, if the "ground contact position" in the fourth layer L4, which is the lowest layer, is identified as the cause, for example, the guidance information for improving it, such as "Let's make the landing position one step closer to yourself.", can be generated.

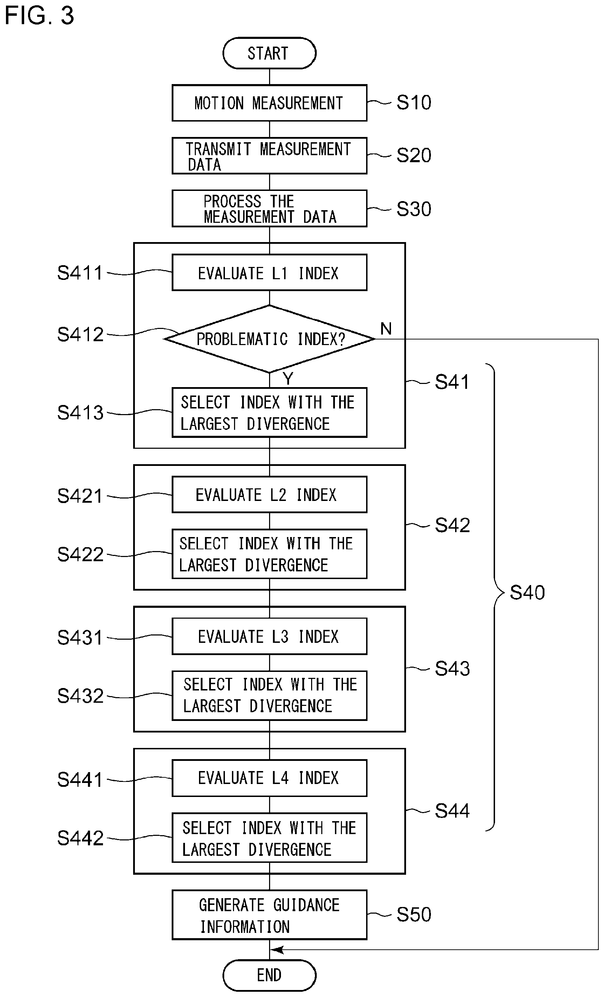

FIG. 3 shows the flow of exercise improvement guidance processing performed by the exercise improvement guidance device 100 using the hierarchical structure as described above. In the following description, "S" means a step.

At S10, measurement by the measurement device 20 is performed during a user's exercise.

At 520, the measurement device 20 transmits the measurement data to the exercise improvement guidance device 100. At the time, when the exercise measurement at S10 is performed using multiple measurement devices 20, the measurement data from the multiple measurement devices 20 may be transmitted to the exercise improvement guidance device 100. Also, when multiple pieces of data are measured using a single measurement device 20, multiple measurement data may be transmitted to the exercise improvement guidance device 100.

At S30, the exercise improvement guidance device 100 processes the various measurement data received at S20 and converts the data into measurement data with which each index in the hierarchical structure can be evaluated. Since many of the indexes in the hierarchical structure cannot be evaluated with the data measured by the measurement device 20 as it is, the data needs to be converted into evaluable data through appropriate arithmetic processing. For the conversion, since various methods are known in the technical field, detailed description thereof will be omitted. With regard to the following indexes described in the flowchart, for example, measurement data for evaluation can be obtained as follows.

The "vertical motion" in the first layer L1 can be directly measured using an acceleration sensor or the like.

The "vertical motion in the air" in the second layer L2 can be measured separately from the vertical motion during ground contact, using an acceleration sensor or the like.

The "pushing-off angle" in the third layer L3 can be calculated based on the measurement data from an acceleration sensor or angular velocity sensor obtained during the transition from the ground contact state to the in-air state.

The "ground contact position" in the fourth layer L4 can be obtained by calculating the relative position of the ground contact position to the takeoff position, based on the measurement data from a position sensor or the like at each of the time of takeoff (ground contact → in-air) and the time of ground contact (in-air → ground contact).

At S40, the problem index identification unit 120 searches for a problem index based on the measurement data processed at S30. The S40 is constituted by S41-S44 corresponding to the four layers L1-L4 of the hierarchical structure. Through the processes of S41, S42, S43, and S44 performed in this order, the search for a problem index is conducted from an upper layer toward a lower layer of the hierarchical structure. More specifically, multiple indexes in each layer are evaluated based on the measurement data, the most problematic index is selected, and the search for the cause is conducted therefrom toward a further lower layer.

The S41, which is a search step for the first layer L1, is constituted by S411-S413.

At S411, multiple indexes in the first layer L1 are evaluated based on the measurement data. In specific, the three indexes of the "landing impact", "vertical motion", and "braking force" in the first layer L1 are evaluated. For each index, reference data representing a normal value thereof is set in advance, and the reference data is compared with the measurement data of the index processed for evaluation at S30, so as to obtain the divergence therebetween. Based on the degree of the divergence, whether or not there is a problem with the index is evaluated. In the following, to simplify the explanation, a criterion for evaluating an index as having a problem is uniformly set to "the divergence of 10% or greater" for all indexes. However, in practice, a different criterion can be set for each index. Also, it is assumed that the degree of the divergence represents urgency, i.e., priority in the exercise improvement guidance, of the index. Therefore, with regard to an index with the divergence of 20% and an index with the divergence of 15%, the former has a higher degree of urgency and corresponds to a priority index in the exercise improvement guidance.

At S412, it is judged whether or not there has been a problematic index at S411. More specifically, with regard to the three indexes of the "landing impact", "vertical motion", and "braking force" in the first layer L1, it is judged whether or not there is an index with the divergence of 10% or greater. If the divergence of each of the indexes is less than 10%, it can be said that all indexes, including the respective lower indexes, fall within a normal range. Accordingly, the problem index identification unit 120 does not identify any problem index, and the guidance information generating unit 130 terminates the process without generating the guidance information. In such a case, the guidance information generating unit 130 may generate a message to motivate the user, such as "Keep up the good work".

If there is an index with the divergence of 10% or greater at S412, on the other hand, the index with the largest divergence will be selected at S413. In the hierarchical structure shown in FIG. 2, when the divergence of the "landing impact" is 15%, the divergence of the "vertical motion" is 20%, and the divergence of the "braking force" is 7%, for example, the "landing impact" and "vertical motion" with the divergences of 10% or greater are problematic, but the "vertical motion" with the largest divergence has a higher degree of urgency, so that the "vertical motion" is selected as a priority index. In the subsequent steps, the search for the problem index is conducted therefrom toward a further lower layer.

The S42 is a search step for the second layer L2, in which a lower index of the priority index "vertical motion" selected at S413 is searched for, and is constituted by S421-S422.

At S421, the multiple indexes of the "vertical motion during ground contact" and "vertical motion in the air" in the second layer L2, which are lower indexes of the priority index "vertical motion" in the first layer L1, are evaluated based on the measurement data. In specific, for each index, predetermined reference data and the measurement data are compared to obtain the divergence therebetween.

At S422, an index with the largest divergence is selected. For example, when the divergence of the "vertical motion during ground contact" is 8% and the divergence of the "vertical motion in the air" is 15%, the "vertical motion in the air" with the largest divergence has a higher degree of urgency, so that the "vertical motion in the air" is selected as a priority index. In the subsequent steps, the search for the problem index is conducted therefrom toward a further lower layer.

The S43 is a search step for the third layer L3, in which a lower index of the priority index "vertical motion in the air" selected at S422 is searched for, and is constituted by S431-S432.

At S431, the multiple indexes of the "pushing-off angle" and "pushing-off acceleration" in the third layer L3, which are lower indexes of the priority index "vertical motion in the air" in the second layer L2, are evaluated based on the measurement data. In specific, for each index, predetermined reference data and the measurement data are compared to obtain the divergence therebetween.

At S432, an index with the largest divergence is selected. For example, when the divergence of the "pushing-off angle" is 17% and the divergence of the "pushing-off acceleration" is 8%, the "pushing-off angle" with the largest divergence has a higher degree of urgency, so that the "pushing-off angle" is selected as a priority index. In the subsequent steps, the search for the problem index is conducted therefrom toward a further lower layer.

The S44 is a search step for the fourth layer L4, in which a lower index of the priority index "pushing-off angle" selected at S432 is searched for, and is constituted by S441-S442.

At S441, the multiple indexes of the "ground contact position", "ground contact angle", and "trunk angle" in the fourth layer L4, which are lower indexes of the priority index "pushing-off angle" in the third layer L3, are evaluated based on the measurement data. In specific, for each index, predetermined reference data and the measurement data are compared to obtain the divergence therebetween.

At S442, an index with the largest divergence is selected. For example, when the divergence of the "ground contact position" is 20%, the divergence of the "ground contact angle" is 13%, and the divergence of the "trunk angle" is 5%, the "ground contact position" with the largest divergence has a higher degree of urgency, so that the "ground contact position" is selected as a priority index. Therefore, the problem index identification unit 120 identifies the selected priority index "ground contact position" as the problem index.

At S50, the guidance information generating unit 130 generates the guidance information, such as "Let's make the landing position one step closer to yourself.", for improving the problem index "ground contact position" identified by the problem index identification unit 120. The guidance information thus generated is transmitted to the display device 30 and displayed on the display screen thereof.

According to the process flow described above, by referring to the relationships among the indexes shown in the hierarchical structure, a problem index that leads to effective improvement of exercise motions can be identified, and the guidance information can be generated.

Since the problem index identification unit 120 searches for the problem index from an upper layer toward a lower layer of the hierarchical structure, a major problem can be identified in an upper layer, and the underlying cause thereof can be identified in a lower layer. Therefore, effective exercise improvement guidance can be provided.

The search for the problem index is performed sequentially from the first layer L1 as the highest layer toward the fourth layer as the lowest layer, and, in the search processes S41, S42, S43, and S44 in the respective layers, a priority index is identified based on the divergence between an actual measured value and a reference value of each index. Accordingly, an index with the highest degree of urgency can be identified in each layer, and an index that needs to be improved on a top-priority basis can be identified as the problem index.

Since the problem index identification unit 120 identifies the problem index in the fourth layer L4 as the lowest layer of the hierarchical structure, a problem index can be identified that represents the underlying cause of the problem and that facilitates the generation of specific guidance information for improvement.

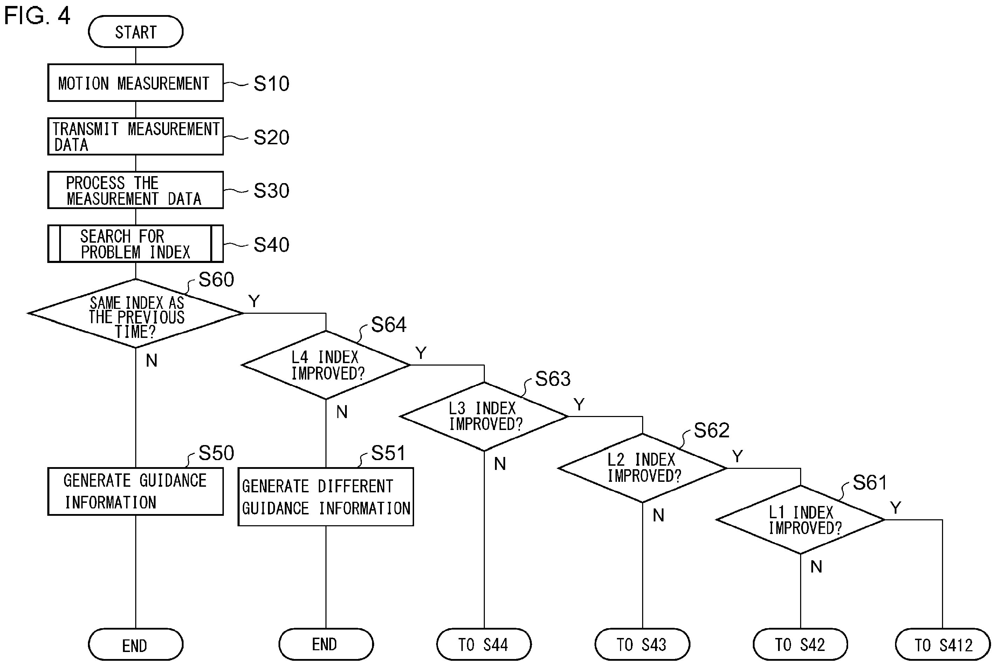

FIG. 4 shows the flow of exercise improvement guidance processing performed by the exercise improvement guidance device 100 when the user performs another running after the exercise improvement guidance according to the process flow shown in FIG. 3 is provided. In this processing, upon identification of an index in which there has been a certain degree of improvement compared to past running, the problem index identification unit 120 searches for a problem index among other indexes present in the same layer as the index or in a higher layer. Also, when the problem index identification unit 120 has identified the same problem index as in past running, the guidance information generating unit 130 generates guidance information that is different from that in the past running. In FIG. 4, the processes of S10, S20, S30, and S40 are performed as in FIG. 3, and, at S442 as the last step in S40, a priority index with a larger divergence is selected. In the following, although the previous running will be described as an example of the past running, the processing in the present embodiment is also applicable to past running prior to the previous running.

At S60, it is judged whether or not the priority index selected at S442 for the current exercise is the same as the priority index "ground contact position" selected at S442 for the previous exercise. When the priority index is different from that of the previous exercise, the problem index identification unit 120 identifies the priority index as the problem index, and the guidance information generating unit 130 generates the guidance information for improving the problem index at S50. For example, when the problem index of the current exercise is the "trunk angle", the guidance information of "Keep your trunk straight." or the like is generated.

Meanwhile, when the priority index is the "ground contact position", which is the same as for the previous exercise, it is judged at S64 whether or not there has been a certain degree of improvement in the index compared to the previous exercise. A criterion for judging whether or not there has been an improvement can be set arbitrarily for each index, and the divergence described previously may also be used. For example, it may be judged that there has been an improvement when the divergence has become smaller than that of the previous exercise by 5% or more. Since the divergence of the "ground contact position" of the previous exercise is 20%, if the divergence of the current exercise is smaller than 15%, it will be judged that there has been a certain degree of improvement.

When it is judged at S64 that there has not been a certain degree of improvement in the "ground contact position", i.e., when the divergence is still high at 15% or greater, the problem index identification unit 120 identifies, as the problem index, the same "ground contact position" as for the previous exercise. In this case, the guidance information generating unit 130 generates again the guidance information for improving the "ground contact position" at S51 but generates different guidance information from the previous one. This allows the user to improve the same problem index from a different perspective.

When it is judged at S64 that there has been a certain degree of improvement in the "ground contact position", i.e., when the divergence has become smaller than 15%, the search is continued with the target layer shifted to the third layer L3 as an upper layer. In specific, at S63, it is judged whether or not there has been a certain degree of improvement compared to the previous exercise, with regard to the "pushing-off angle" (the third layer L3), which is the upper index of the "ground contact position" (the fourth layer L4).

When it is judged at S63 that there has not been a certain degree of improvement in the "pushing-off angle", it means that the problem index to be preferentially improved is still present in the lower layer. Accordingly, the process returns to S44 in FIG. 3, and the search in the lower layer (the fourth layer L4) is continued. At the time, since the divergence of the "ground contact position" is the largest in the fourth layer L4, if S442 in S44 is performed ordinarily, the "ground contact position" will eventually be identified as the problem index. However, since the "ground contact position" has been improved to a certain degree compared to the previous exercise (S64), it may be excluded from the candidates for the problem index of the current exercise. In such a case, at S442, the index with the largest divergence among the other indexes "ground contact angle" and "trunk angle", excluding the "ground contact position", will be identified as the problem index. Meanwhile, S442 may be performed ordinarily, and the same "ground contact position" as for the previous exercise may be identified as the problem index. In such a case, as described in relation to S51, guidance information different from the previous one may preferably be generated.

When it is judged at S63 that there has been a certain degree of improvement in the "pushing-off angle", the search is continued with the target layer shifted to the second layer L2 as an upper layer. In specific, at S62, it is judged whether or not there has been a certain degree of improvement compared to the previous exercise, with regard to the "vertical motion in the air" (the second layer L2), which is the upper index of the "pushing-off angle" (the third layer L3).

When it is judged at S62 that there has not been a certain degree of improvement in the "vertical motion in the air", it means that the problem index to be preferentially improved is still present in the lower layer. Accordingly, the process returns to S43 in FIG. 3, and the search in the lower layer (the third layer L3) is continued.

When it is judged at S62 that there has been a certain degree of improvement in the "vertical motion in the air", the search is continued with the target layer shifted to the first layer L1 as the upper layer. In specific, at S61, it is judged whether or not there has been a certain degree of improvement compared to the previous exercise, with regard to the "vertical motion" (the first layer L1), which is the upper index of the "vertical motion in the air" (the second layer L2).

When it is judged at S61 that there has not been a certain degree of improvement in the "vertical motion", it means that the problem index to be preferentially improved is still present in the lower layer. Accordingly, the process returns to S42 in FIG. 3, and the search in the lower layer (the second layer L2) is continued.

When it is judged at S61 that there has been a certain degree of improvement in the "vertical motion", the process returns to S412 in FIG. 3 to judge whether or not there is a problematic index in the first layer L1. At the time, since the "vertical motion" has been improved to a certain degree compared to the previous exercise, it may preferably be excluded from the judgment targets at S412. In such a case, at S412, the judgment is performed for the other indexes "landing impact" and "braking force", excluding the "vertical motion".

According to the process flow described above, the problem index of the current exercise can be efficiently searched for, also in consideration of improvement from a past exercise. At the time, through the improvement judgment processes S64, S63, S62, and S61 performed sequentially from a lower layer toward an upper layer of the hierarchical structure, in which layer there has been an improvement can be efficiently identified.

The present invention has been described based on an embodiment. The embodiment is intended to be illustrative only, and it will be obvious to those skilled in the art that various modifications to a combination of constituting elements or processes could be developed and that such modifications also fall within the scope of the present invention.

In the embodiment described above, the search for the problem index by the problem index identification unit 120 is performed from an upper layer toward a lower layer of the hierarchical structure. However, the method for searching the hierarchical structure is not limited thereto. For example, instead of starting the search for the problem index from the highest layer, the search may be started from a layer specified in advance by the user or the system. Also, the lowest layer may be searched at the beginning, and the index with the largest divergence in the layer may be identified as the problem index. In this search, the indexes in the layers other than the lowest layer are not considered. However, when exercise improvement guidance is provided also in consideration of improvement from a past exercise, as described with reference to FIG. 4, the indexes in each layer are considered, as shown in S64, S63, S62, and S61.

Also, the abovementioned embodiment describes an example in which every index in the highest layer L1 is subdivided into indexes in the lowest layer L4, and the depth of the hierarchical structure is uniformly four layers. However, the depth of the hierarchical structure may be different depending on each index. More specifically, when the highest layer is defined as L1, as in the embodiment, there may be an index with a depth 1 that has no hierarchical structure below the first layer L1, there may be an index with a depth 2 that is subdivided into indexes in the second layer L2, and there may be an index with a depth 3 that is subdivided into indexes in the third layer L3. At the time, not all layers need to include corresponding indexes. For example, indexes resulting from subdivision of one index in the first layer L1 may not be present in the second layer L2 immediately below, but the one index may be subdivided into indexes in the third layer L3, which is further lower. Also, the highest layer of every index need not necessarily be the first layer L1, and the highest index may be present in the second layer L2, the third layer L3, or the fourth layer L4. When the hierarchical structure with different depths as described above is searched, the search need not necessarily be performed to the lowest layer, and the depth to be searched may be specified each time by the user or the system for each index. For example, even when a hierarchical structure with the depth 3 is to be searched, if the search to the depth 2 is specified, the lowest layer of the depth 3 will not be searched, and the problem index is identified in the layer of the depth 2 thereabove.

In the abovementioned embodiment, the problem index is identified in the lowest layer of the hierarchical structure. However, the problem index may be identified in the other layers. For example, an index with the largest divergence in all layers may be identified as the problem index. In this case, it is preferable to prepare the guidance information for all indexes regardless of the layers. Also, when the guidance information for an index in a layer has been generated for a past exercise and if, as a result, the index in the layer has been sufficiently improved in the current exercise, the problem index may be identified in an upper layer. Accordingly, in consideration of the improvement of the user's exercise motions, guidance based on an appropriate layer can be provided.

In the embodiment described above, during the search for the problem index by the problem index identification unit 120, an index with the largest divergence between the reference data and the measurement data is selected in each layer, and the search for the problem index is performed toward a further lower layer. However, multiple indexes of which the divergences are larger than a preset value may be selected in each layer. In such a case, the lower layers of each of the selected indexes are searched to identify multiple problem indexes. The guidance information generating unit 130 then generates the guidance information for each problem index thus identified and displays the guidance information on the display device 30. On the display device 30, the multiple pieces of guidance information may be displayed on a single screen, or multiple screens may be switched by user operation so that the guidance information for each problem index can be checked.

In the process flow shown in FIG. 4, when an index in a layer has been improved to a certain degree compared to a past exercise, it is judged whether or not an index in the upper layer has been improved. However, it may also be judged whether or not another index in the same layer has been improved. For example, in FIG. 4, when it is judged at S64 that the "ground contact position" in the fourth layer L4 has been improved, it is judged at S63 whether or not the "pushing-off angle" in the third layer L3 has been improved. Instead, it may be judged whether or not the other indexes "ground contact angle" and "trunk angle" in the fourth layer L4 have been improved. Since these indexes in the fourth layer L4 are subordinate to the "pushing-off angle" in the third layer L3, there is no significant difference in judging whether or not there has been an improvement in either layer.

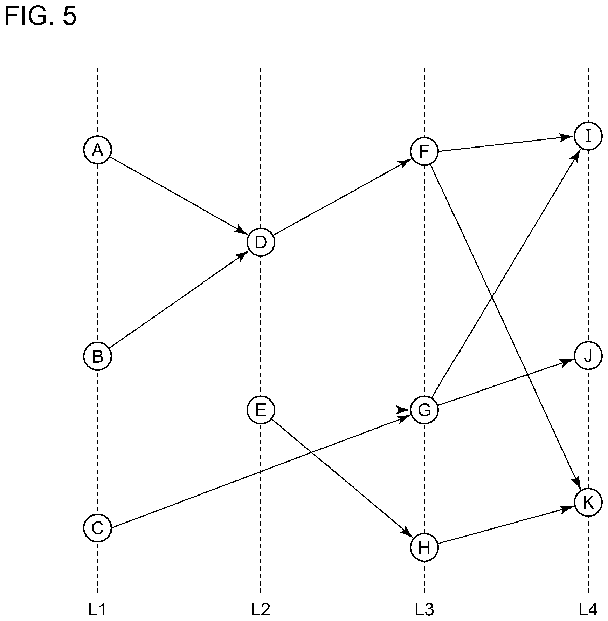

The abovementioned embodiment describes, as an example of the hierarchical structure, a tree structure as shown in FIG. 2, i.e., a structure that branches from an upper layer toward a lower layer. However, the hierarchical structure in the present invention is not limited to such a tree structure. FIG. 5 shows another example of the hierarchical structure in the form of a directed graph. In FIG. 5, the vertices A-K of the directed graph represent the indexes in the layers L1-L4, and each of the directed edges connecting the indexes indicates the direction of the search for a problem index. In the first layer L1 as the highest layer, there are indexes A, B, and C.

In the second layer L2, there are indexes D and E. The index D is connected with edges (arrows) starting respectively from the indexes A and B in the first layer L1, which means that the index D is evaluated when the divergences of the indexes A and B are larger than a predetermined value. Therefore, if at least one of the divergences of the indexes A and B is equal to or less than the predetermined value, the search for the problem index with regard to the indexes A and B will be terminated in the first layer L1. At the time, if the divergences of both the indexes A and B are equal to or less than the predetermined value, none of them is the problem index; if one of the divergences of the indexes A and B is larger than the predetermined value, it is identified as the problem index. Although similar description will be omitted hereafter, a directed edge (arrow) means that, when the divergence is larger than a predetermined value in this way, the process proceeds to the search for an index in the lower layer. Unlike the tree structure in FIG. 2, in this example, it can also be construed that the upper indexes A and B are integrated into the lower index D.

In the third layer L3, there are indexes F, G, and H. The index F is designated by an arrow from the index D in the second layer L2. The index G is designated by arrows from the index E in the second layer L2 and the index C in the first layer L1. The index H is designated by an arrow from the index E in the second layer L2.

In the fourth layer L4, there are indexes I, J, and K. The index I is designated by arrows from the indexes F and G in the third layer L3. The index J is designated by an arrow from the index G in the third layer L3. The index K is designated by arrows from the indexes F and H in the third layer L3.

With the hierarchical structure using a directed graph as described above, complicated relationships among indexes can be described more appropriately than with the tree structure shown in FIG. 2, so that a problem index can be effectively identified. Also, although only the arrows directed from upper layers to lower layers are illustrated in FIG. 5, the processing for searching for improvement from a past exercise from a lower layer toward an upper layer as described with reference to FIG. 4 can be described with arrows directed from indexes in lower layers to indexes in upper layers in FIG. 5.

The present embodiment has been described employing running as an example of the exercise. However, the present invention is also applicable to other exercises. For example, it is applicable to various athletics, swimming, gymnastics, walking, training and exercises for road biking or the like, dance, and ball sports such as soccer.

The functional configuration of each device described in the embodiment can be implemented by hardware resources, software resources, or cooperation between hardware resources and software resources. As the hardware resources, processors, ROMs, RAMs, or other LSIs can be employed. As the software resources, programs, such as operating system programs and application programs, can be employed.

### REFERENCE SIGNS LIST

- 100: exercise improvement guidance device
- 110: hierarchical index storage unit
- 120: problem index identification unit
- 130: guidance information generating unit
- 20: measurement device
- 30: display device

### INDUSTRIAL APPLICABILITY

The present invention relates to an exercise improvement guidance device for identifying problems for improvement of exercise motions.

## Claims

1. An exercise improvement guidance device (100), comprising:
a hierarchical index storage unit (110) that is configured to store, in layers, hierarchized indexes for improvement of an exercise motion by a user, wherein the device is configured to evaluate the indexes based on measurement data;
wherein each of these indexes in a higher layer of the layers is subdivided toward a lower layer, and the hierarchy is such that causes of user exercise problems represented by indexes in higher of the layers are identifiable from indexes in lower of the layers; and
a problem index identification unit (120) that is configured to evaluate the hierarchized indexes in a first layer based on measurement data during an exercise and is configured to identifz a priority index in the first layer of which a divergence between predetermined reference data and the measurement data is larger than a preset value, wherein
the measurement data is associated with the user's exercise motions,
wherein the problem index identification unit is configured to sequentially search from the first layer towards a lowest layer whereby:
a respective priority index is identified at each layer and for each layer the search is then conducted from the identified priority index towards a next lower layer,
for each searched layer the problem index identification unit (120) performs a respective search process that comprises searching amongst indexes into which the identified priority index of the layer above is sub-divided,
and for each searched layer the respective search process comprises identifying indexes of said sub-divided indexes of that layer for which a divergence between predetermined reference data and the measurement data is larger than a preset value, and selecting a respective priority index for that layer based on the degree of divergence identified for said sub-divided indexes,
wherein the problem index identification unit (120) is configured to identify as the problem index the priority index selected by the search process for the lowest specified layer of the hierarchical structure of the indexes.

2. The exercise improvement guidance device (100) according to claim 1, further comprising a guidance information generating unit (130) that is configured to generate guidance information for improvement of the problem index identified by the problem index identification unit (120).

3. The exercise improvement guidance device (100) according to claim 1 or 2, wherein the problem index identification unit (120) is configured to search for the problem index from a pre-specified layer of a hierarchical structure of the indexes.

4. The exercise improvement guidance device (100) according to any one of claims 1 through 3, wherein the problem index identification unit (120) is configured to identify the problem index in the lowest layer of the hierarchical structure of the indexes.

5. The exercise improvement guidance device (100) according to any one of claims 1 through 4, wherein, upon identification of an index in which there has been a predetermined degree of improvement compared to a past exercise, the problem index identification unit (120) is configured to search for the problem index among other indexes present in the same layer as the index or in a higher layer.

6. The exercise improvement guidance device (100) according to claim 2, wherein, when the problem index identification unit (120) has identified the same problem index as in a past exercise, the guidance information generating unit (130) is configured to generate the guidance information that is different from that in the past exercise.

7. An exercise improvement guidance method, comprising:
a hierarchical index storing step of storing, in layers, hierarchized indexes for improvement of an exercise motion by a user, wherein the indexes are evaluated based on measurement data;
wherein each of these indexes in a higher layer of the layers is subdivided toward a lower layer, and the hierarchy is such that causes of user exercise problems represented by indexes in higher of the layers are identifiable from indexes in lower of the layers; and
a problem index identification step of evaluating the hierarchized indexes in a first layer based on measurement data during an exercise and identifying a priority index in the first layer of which a divergence between predetermined reference data and the measurement data is larger than a preset value, wherein
the measurement data is associated with the user's exercise motions,
wherein the problem index identification step comprises sequentially searching from the first layer towards a lowest layer and a respective priority index is identified at each layer and for each layer the search is then conducted from the identified priority index towards a next lower layer,
for each searched layer the problem index identification step comprises performing a respective search process that comprises searching amongst indexes into which the identified priority index of the layer above is sub-divided,
and for each searched layer the respective search process comprises identifying indexes of said sub-divided indexes of that layer for which a divergence between predetermined reference data and the measurement data is larger than a preset value, and selecting a respective priority index for that layer based on the degree of divergence identified for said sub-divided indexes,
wherein the problem index identification step comprises identifying as the problem index the priority index selected by the search process for the lowest specified layer of the hierarchical structure of the indexes.

8. An exercise improvement guidance program causing a computer to implement:
a hierarchical index storing step of storing, in layers, hierarchized indexes for improvement of an exercise motion by a user, wherein the indexes are evaluated based on measurement data;
wherein each of these indexes in a higher layer of the layers is subdivided toward a lower layer, and the hierarchy is such that causes of user exercise problems represented by indexes in higher of the layers are identifiable from indexes in lower of the layers; and
a problem index identification step of evaluating the hierarchized indexes in a first layer based on measurement data during an exercise and identifying a priority index in the first layer of which a divergence between predetermined reference data and the measurement data is larger than a preset value, wherein
the measurement data is associated with the user's exercise motions,
wherein the problem index identification step comprises sequentially searching from the first layer towards a lowest layer and a respective priority index is identified at each layer and for each layer the search is then conducted from the identified priority index towards a next lower layer,
for each searched layer the problem index identification step comprises performing a respective search process that comprises searching amongst indexes into which the identified priority index of the layer above is sub-divided,
and for each searched layer the respective search process comprises identifying indexes of said sub-divided indexes of that layer for which a divergence between predetermined reference data and the measurement data is larger than a preset value, and selecting a respective priority index for that layer based on the degree of divergence identified for said sub-divided indexes,
wherein the problem index identification step comprises identifying as the problem index the priority index selected by the search process for the lowest specified layer of the hierarchical structure of the indexes.

## Patentansprüche

1. Übungsverbesserungsanleitungsvorrichtung (100), umfassend:
eine hierarchische Indexspeichereinheit (110), die konfiguriert ist, um hierarchisierte Indizes zur Verbesserung einer Übungsbewegung durch einen Benutzer in Schichten zu speichern, wobei die Vorrichtung konfiguriert ist, um die Indizes basierend auf Messdaten auszuwerten;
wobei jeder dieser Indizes in einer höheren Schicht der Schichten in Richtung einer niedrigeren Schicht unterteilt ist und die Hierarchie so beschaffen ist, dass Ursachen von Benutzerübungsproblemen, die durch Indizes in höheren der Schichten dargestellt sind, aus Indizes in niedrigeren der Schichten identifizierbar sind; und
eine Problemindex-Identifizierungseinheit (120), die konfiguriert ist, um die hierarchisierten Indizes einer ersten Schicht basierend auf Messdaten während einer Übung auszuwerten und konfiguriert ist, um einen Prioritätsindex in der ersten Schicht zu identifizieren, bei dem eine Abweichung zwischen vorbestimmten Referenzdaten und den Messdaten größer als ein voreingestellter Wert ist, wobei
die Messdaten den Übungsbewegungen des Benutzers zugeordnet sind, wobei die Problemindex-Identifizierungseinheit konfiguriert ist, um sequenziell von der ersten Schicht in Richtung der niedrigsten Schicht zu suchen, wodurch:
in jeder Schicht ein entsprechender Prioritätsindex identifiziert wird, und die Suche danach für jede Schicht vom ermittelten Prioritätsindex in Richtung einer nächstniedrigeren Schicht durchgeführt wird,
die Problemindex-Identifizierungseinheit (120) für jede durchsuchte Schicht einen entsprechenden Suchprozess durchführt, der Suchen zwischen Indizes umfasst, in die der identifizierte Prioritätsindex der darüber liegenden Schicht unterteilt ist,
und der jeweilige Suchprozess für jede durchsuchte Schicht Identifizieren von Indizes der unterteilten Indizes dieser Schicht umfasst, bei denen eine Abweichung zwischen vorbestimmten Referenzdaten und den Messdaten größer als ein voreingestellter Wert ist, und Auswählen eines jeweiligen Prioritätsindex für diese Schicht basierend auf dem Abweichungsgrad, der für die unterteilten Indizes identifiziert worden ist,
wobei die Problemindex-Identifizierungseinheit (120) konfiguriert ist, um den vom Suchprozess für die niedrigste angegebene Schicht der hierarchischen Struktur der Indizes ausgewählten Prioritätsindex als Problemindex zu identifizieren.

2. Übungsverbesserungsanleitungsvorrichtung (100) nach Anspruch 1, weiter umfassend eine Anleitungsinformationsgenerierungseinheit (130), die konfiguriert ist, um Anleitungsinformationen zum Verbessern des von der Problemindex-Identifizierungseinheit (120) identifizierten Problemindex zu generieren.

3. Übungsverbesserungsanleitungsvorrichtung (100) nach Anspruch 1 oder 2, wobei die Problemindex-Identifizierungseinheit (120) konfiguriert ist, um aus einer vordefinierten Schicht einer hierarchischen Struktur der Indizes nach dem Problemindex zu suchen.

4. Übungsverbesserungsanleitungsvorrichtung (100) nach einem der Ansprüche 1 bis 3, wobei die Problemindex-Identifizierungseinheit (120) konfiguriert ist, um in einer niedrigsten Schicht der hierarchischen Struktur der Indizes den Problemindex zu identifizieren.

5. Übungsverbesserungsanleitungsvorrichtung (100) nach einem der Ansprüche 1 bis 4, wobei beim Identifizieren eines Index, bei dem im Vergleich zu einer früheren Übung ein vorbestimmter Verbesserungsgrad gewesen ist, die Problemindex-Identifizierungseinheit (120) konfiguriert ist, um unter anderen Indizes, die in derselben Schicht wie der Index oder in einer höheren Schicht vorhanden sind, nach dem Problemindex zu suchen.

6. Übungsverbesserungsanleitungsvorrichtung (100) nach Anspruch 2, wobei, wenn die Problemindex-Identifizierungseinheit (120) denselben Problemindex wie in einer früheren Übung identifiziert hat, die Anleitungsinformationsgenerierungseinheit (130) konfiguriert ist, um die Anleitungsinformation zu generieren, die sich von derjenigen in der früheren Übung unterscheidet.

7. Übungsverbesserungsanleitungsverfahren, umfassend:
einen hierarchischen Indexspeicherungsschritt zum Speichern in Schichten von hierarchisierten Indizes zur Verbesserung einer Übungsbewegung durch einen Benutzer, wobei die Indizes basierend auf Messdaten ausgewertet werden;
wobei jeder dieser Indizes in einer höheren Schicht der Schichten in Richtung einer niedrigeren Schicht unterteilt ist und die Hierarchie so beschaffen ist, dass Ursachen von Benutzerübungsproblemen, die durch Indizes in höheren der Schichten dargestellt sind, aus Indizes in niedrigeren der Schichten identifizierbar sind; und
einen Problemindex-Identifizierungsschritt zum Bewerten der hierarchisierten Indizes einer ersten Schicht basierend auf Messdaten während einer Übung und Identifizieren eines Prioritätsindex in der ersten Schicht, bei dem eine Abweichung zwischen vorbestimmten Referenzdaten und den Messdaten größer als ein voreingestellter Wert ist, wobei
die Messdaten den Übungsbewegungen des Benutzers zugeordnet sind,
wobei der Problemindex-Identifizierungsschritt sequenzielles Suchen von der ersten Schicht in Richtung der niedrigsten Schicht umfasst und in jeder Schicht wird ein entsprechender Prioritätsindex identifiziert wird, und die Suche danach für jede Schicht vom ermittelten Prioritätsindex in Richtung einer nächstniedrigeren Schicht durchgeführt wird,
der Problemindex-Identifizierungsschritt für jede durchsuchte Schicht Durchführen eines entsprechenden Suchprozesses umfasst, der Suchen zwischen Indizes umfasst, in die der identifizierte Prioritätsindex der darüber liegenden Schicht unterteilt ist,
und der jeweilige Suchprozess für jede durchsuchte Schicht Identifizieren von Indizes der unterteilten Indizes dieser Schicht umfasst, bei denen eine Abweichung zwischen vorbestimmten Referenzdaten und den Messdaten größer als ein voreingestellter Wert ist, und Auswählen eines jeweiligen Prioritätsindex für diese Schicht basierend auf dem Abweichungsgrad, der für die unterteilten Indizes identifiziert worden ist,
wobei der Problemindex-Identifizierungsschritt Identifizieren des vom Suchprozess für die niedrigste angegebene Schicht der hierarchischen Struktur der Indizes ausgewählten Prioritätsindex als Problemindex umfasst.

8. Übungsverbesserungsanleitungsprogramm, das einen Computer veranlasst, zu implementieren:
einen hierarchischen Indexspeicherungsschritt zum Speichern in Schichten von hierarchisierten Indizes zur Verbesserung einer Übungsbewegung durch einen Benutzer, wobei die Indizes basierend auf Messdaten ausgewertet werden;
wobei jeder dieser Indizes in einer höheren Schicht der Schichten in Richtung einer niedrigeren Schicht unterteilt ist und die Hierarchie so beschaffen ist, dass Ursachen von Benutzerübungsproblemen, die durch Indizes in höheren der Schichten dargestellt sind, aus Indizes in niedrigeren der Schichten identifizierbar sind; und
einen Problemindex-Identifizierungsschritt zum Bewerten der hierarchisierten Indizes einer ersten Schicht basierend auf Messdaten während einer Übung und Identifizieren eines Prioritätsindex in der ersten Schicht, bei dem eine Abweichung zwischen vorbestimmten Referenzdaten und den Messdaten größer als ein voreingestellter Wert ist, wobei
die Messdaten den Übungsbewegungen des Benutzers zugeordnet sind,
wobei der Problemindex-Identifizierungsschritt sequenzielles Suchen von der ersten Schicht in Richtung der niedrigsten Schicht umfasst und in jeder Schicht wird ein entsprechender Prioritätsindex identifiziert wird, und die Suche danach für jede Schicht vom ermittelten Prioritätsindex in Richtung einer nächstniedrigeren Schicht durchgeführt wird,
der Problemindex-Identifizierungsschritt für jede durchsuchte Schicht Durchführen eines entsprechenden Suchprozesses umfasst, der Suchen zwischen Indizes umfasst, in die der identifizierte Prioritätsindex der darüber liegenden Schicht unterteilt ist,
und der jeweilige Suchprozess für jede durchsuchte Schicht Identifizieren von Indizes der unterteilten Indizes dieser Schicht umfasst, bei denen eine Abweichung zwischen vorbestimmten Referenzdaten und den Messdaten größer als ein voreingestellter Wert ist, und Auswählen eines jeweiligen Prioritätsindex für diese Schicht basierend auf dem Abweichungsgrad, der für die unterteilten Indizes identifiziert worden ist,
wobei der Problemindex-Identifizierungsschritt Identifizieren des vom Suchprozess für die niedrigste angegebene Schicht der hierarchischen Struktur der Indizes ausgewählten Prioritätsindex als Problemindex umfasst.

## Revendications

1. Dispositif d'instruction pour l'optimisation d'exercice (100), comprenant :
une unité de stockage d'indice hiérarchique (110) qui est configurée pour stocker, en couches, des indices hiérarchisés pour l'optimisation d'un mouvement d'exercice par un utilisateur, dans laquelle le dispositif est configuré pour évaluer les indices sur la base de données de mesure ;
dans lequel chacun de ces indices d'une couche supérieure est subdivisé vers une couche inférieure, et la hiérarchie est telle que les causes des problèmes d'exercice d'utilisateur représentées par les indices des couches supérieures sont identifiables à partir des indices des couches inférieures ; et
une unité d'identification d'indice de problème (120) qui est configurée pour évaluer les indices hiérarchisés dans une première couche sur la base de données de mesure lors d'un exercice, et configurée pour identifier un indice prioritaire dans la première couche dont l'écart entre les données de référence prédéterminées et les données de mesure est supérieur à une valeur prédéfinie, dans laquelle
les données de mesure sont associées aux mouvements d'exercice d'utilisateur, dans lesquels l'unité d'identification de l'indice de problème est configurée pour effectuer une recherche séquentielle de la première couche vers la couche la plus basse, de sorte que :
un indice de priorité respectif est identifié à chaque couche et, pour chaque couche, la recherche est ensuite menée à partir de l'indice de priorité identifié vers la couche inférieure suivante,
pour chaque couche recherchée, l'unité d'identification d'indice de problème (120) effectue un processus de recherche respectif qui consiste à rechercher parmi les indices dans lesquels l'indice de priorité identifié de la couche supérieure est subdivisé,
et pour chaque couche recherchée, le processus de recherche respectif comprend l'identification des indices desdits sous-indices de cette couche pour lesquels une divergence entre les données de référence prédéterminées et les données de mesure est supérieure à une valeur prédéfinie, et la sélection d'un indice prioritaire respectif pour cette couche basée sur le degré de divergence identifié pour lesdits sous-indices,
dans lequel l'unité d'identification de l'indice du problème (120) est configurée pour identifier comme indice de problème l'indice prioritaire sélectionné par le processus de recherche pour la couche spécifiée la plus basse de la structure hiérarchique des indices.

2. Dispositif de guidage d'amélioration d'exercice (100) selon la revendication 1, comprenant en outre une unité de génération d'informations de guidage (130) qui est configurée pour générer des informations de guidage pour l'amélioration de l'indice de problème identifié par l'unité d'identification de l'indice de problème (120).

3. Dispositif de guidage d'amélioration de l'exercice (100) selon la revendication 1 ou 2, dans lequel l'unité d'identification de l'indice de problème (120) est configurée pour rechercher l'indice de problème à partir d'une couche prédéfinie d'une structure hiérarchique des indices.

4. Dispositif de guidage d'amélioration de l'exercice (100) selon l'une quelconque des revendication 1 à 3, dans lequel l'unité d'identification de l'indice de problème (120) est configurée pour identifier l'indice de problème à partir d'une couche prédéfinie d'une structure hiérarchique des indices.

5. Dispositif de guidage d'amélioration d'exercice (100) selon l'une quelconque des revendications 1 à 4, dans lequel, lors de l'identification d'un indice dans lequel il y a eu un degré d'amélioration prédéterminé par rapport à un exercice antérieur, l'unité d'identification d'indice de problème (120) est configurée pour rechercher l'indice de problème parmi d'autres indices présents dans la même couche que l'indice ou dans une couche supérieure.

6. Dispositif de guidage d'amélioration d'exercice (100) selon la revendication 2, dans lequel, lorsque l'unité d'identification d'indice de problème (120) a identifié le même indice de problème que dans un exercice précédent, l'unité de génération d'informations de guidage (130) est configurée pour générer des informations de guidage différentes de celles de l'exercice précédent.

7. Procédé d'instruction pour l'optimisation d'exercice, comprenant :
une étape de stockage d'indice hiérarchique consistant à stocker, en couches, des indices hiérarchisés pour l'optimisation d'un mouvement d'exercice par un utilisateur, dans laquelle les indices sont évalués sur la base de données de mesure ;
dans lequel chacun de ces indices d'une couche supérieure est subdivisé vers une couche inférieure, et la hiérarchie est telle que les causes des problèmes d'exercice d'utilisateur représentées par les indices des couches supérieures sont identifiables à partir des indices des couches inférieures ; et
une étape d'identification d'un indice de problème consistant à évaluer les indices hiérarchisés d'une première couche à partir de données de mesure lors d'un exercice et à identifier un indice prioritaire dans la première couche dont l'écart entre les données de référence prédéterminées et les données de mesure est supérieur à une valeur prédéfinie, dans laquelle
les données de mesure sont associées aux mouvements d'exercice de l'utilisateur,
dans laquelle l'étape d'identification de l'indice du problème comprend une recherche séquentielle de la première couche vers une couche la plus basse et un indice de priorité respectif est identifié à chaque couche et, pour chaque couche, la recherche est ensuite menée à partir de l'indice de priorité identifié vers la couche inférieure suivante,
pour chaque couche recherchée, l'étape d'identification d'indice de problème effectue un processus de recherche respectif qui consiste à rechercher parmi les indices dans lesquels l'indice de priorité identifié de la couche supérieure est subdivisé,
et pour chaque couche recherchée, le processus de recherche respectif comprend l'identification des indices desdits sous-indices de cette couche pour lesquels une divergence entre les données de référence prédéterminées et les données de mesure est supérieure à une valeur prédéfinie, et la sélection d'un indice prioritaire respectif pour cette couche basée sur le degré de divergence identifié pour lesdits sous-indices,
dans lequel l'étape d'identification de l'indice du problème est configurée pour identifier comme indice du problème l'indice prioritaire sélectionné par le processus de recherche pour la couche spécifiée la plus basse de la structure hiérarchique des indices.

8. Programme d'accompagnement pour l'optimisation d'exercice qui amène un ordinateur à mettre en œuvre :
une étape de stockage d'indice hiérarchique consistant à stocker, en couches, des indices hiérarchisés pour l'optimisation d'un mouvement d'exercice par un utilisateur, dans laquelle les indices sont évalués sur la base de données de mesure ;
dans lequel chacun de ces indices d'une couche supérieure est subdivisé vers une couche inférieure, et la hiérarchie est telle que les causes des problèmes d'exercice d'utilisateur représentées par les indices des couches supérieures sont identifiables à partir des indices des couches inférieures ; et
une étape d'identification d'un indice de problème consistant à évaluer les indices hiérarchisés d'une première couche à partir de données de mesure lors d'un exercice et à identifier un indice prioritaire dans la première couche dont l'écart entre les données de référence prédéterminées et les données de mesure est supérieur à une valeur prédéfinie, dans laquelle
les données de mesure sont associées aux mouvements d'exercice de l'utilisateur,
dans laquelle l'étape d'identification de l'indice du problème comprend une recherche séquentielle de la première couche vers une couche la plus basse et un indice de priorité respectif est identifié à chaque couche et, pour chaque couche, la recherche est ensuite menée à partir de l'indice de priorité identifié vers la couche inférieure suivante,
pour chaque couche recherchée, l'étape d'identification d'indice de problème effectue un processus de recherche respectif qui consiste à rechercher parmi les indices dans lesquels l'indice de priorité identifié de la couche supérieure est subdivisé,
et pour chaque couche recherchée, le processus de recherche respectif comprend l'identification des indices desdits sous-indices de cette couche pour lesquels une divergence entre les données de référence prédéterminées et les données de mesure est supérieure à une valeur prédéfinie, et la sélection d'un indice prioritaire respectif pour cette couche basée sur le degré de divergence identifié pour lesdits sous-indices,
dans lequel l'étape d'identification de l'indice du problème est configurée pour identifier comme indice du problème l'indice prioritaire sélectionné par le processus de recherche pour la couche spécifiée la plus basse de la structure hiérarchique des indices.
